# EUROPEAN PATENT APPLICATION

(11) **EP 4 202 046 A1**
(43) Date of publication of application: **28.06.2023**
(21) Application number: 21857433.3
(22) Date of filing: 15.07.2021
(51) Int. Cl.: C12N 15/113, C12N 15/77, C12N 15/53, C12N 1/21, C12P 13/08, C12R 1/15

(54) **POLYNUCLEOTIDE HAVING PROMOTER ACTIVITY AND APPLICATION OF POLYNUCLEOTIDE IN PRODUCING AMINO ACID**

(30) Priority: 19.08.2020 CN 202010838604
(71) Applicant: Tianjin Institute of Industrial Biotechnology, Chinese Academy of Sciences, Tianjin Airport Economic Area Tianjin 300308 (CN)
(72) Inventor: ZHENG, Ping, Tianjin 300308 (CN); LIU, Jiao, Tianjin 300308 (CN); SUN, Jibin, Tianjin 300308 (CN); ZHOU, Wenjuan, Tianjin 300308 (CN); SHI, Tuo, Tianjin 300308 (CN); GUO, Xuan, Tianjin 300308 (CN); MA, Yanhe, Tianjin 300308 (CN)
(74) Representative: Heubeck, Christian
(86) International application number: PCT/CN2021/106501
(87) International publication number: WO 2022/037338

(57) **Abstract**

Provided are a polynucleotide having promoter activity and an application of the polynucleotide in producing an amino acid. Also disclosed are a transcription expression cassette, a recombinant expression vector, and a recombinant host cell which contain the polynucleotide, and a method for enhancing expression of a target gene, a method for preparing a protein, and a method for producing an amino acid. The polynucleotide having the promoter activity is a mutant of a polynucleotide having the sequence as shown in SEQ ID NO: 9. Compared with the polynucleotide having the sequence as shown in SEQ ID NO: 9, the promoter activity of the mutant is significantly enhanced, thereby promoting the stable and efficient expression of the target gene.

## Description

### TECHNICAL FIELD

The present disclosure pertains to the field of molecular biology and bioengineering, and particularly relates to a polynucleotide having promoter activity, a transcription expression cassette, a recombinant expression vector and a recombinant host cell comprising the polynucleotide having promoter activity, as well as a method for enhancing the expression of a target gene, a method for preparing proteins and a method for producing amino acids.

### BACKGROUND

Lysine, the chemical name of which is 2,6-diaminopimelic acid, is an essential amino acid for animals and humans. It can promote human development, enhance immunity and has an effect of improving the function of central nervous system. There are three chemical optical isomers of lysine: L-form (levorotary), D-form (dextral) and DL-form (racemic form), among which only L-form is biologically utilizable, and the commonly referred lysine is L-lysine.

L-lysine is one of the 20 common amino acids that make up proteins. L-lysine is a basic amino acid, like histidine and arginine. Since the human bodies and the animal bodies cannot synthesize L-lysine by themselves, L-lysine can only be obtained from food, thus is one of the eight essential amino acids. The content of L-lysine in human staple cereal food is low, and its deficiency will cause metabolic and functional disorders of protein, adversely affecting growth, and is liable to be destroyed during processing, thus is known as the first-limiting amino acid, which plays a very important role in medicine, health, food, animal feed and cosmetics industries.

Up to now, there are three main methods for industrial production of L-lysine: proteolysis, chemical synthesis and microbial fermentation. Among them, microbial fermentation is the most widely used method for industrial production of L-lysine because of its advantages such as low production cost, high production intensity, high specificity and low environmental pollution. *Corynebacterium* and *Escherichia* have been widely used in the industrial production of L-lysine. Commonly used *Escherichia* is, for example, *Escherichia coli,* and commonly used *Corynebacterium* includes *Corynebacterium glutamicum* of genus *Corynebacterium, Brevibacterium flavum* of genus *Brevibacterium, Brevibacterium lactofermentus,* and some species of genus *Arthrobacterium* and some species of genus *Microbacterium.*

Among the microorganisms and plants known to have L-lysine biosynthesis pathways, L-lysine biosynthesis pathways can be classified as two distinct pathways, i.e. alpha-aminoglycolic acid (AAA) pathway and diaminopimelate (DAP) pathway. The diaminopimelate (DAP) pathway is a part of the synthesis pathway of amino acids of the aspartic acid family. Using aspartic acid as the substrate, the DAP pathway includes succinylase pathway, acetylase pathway and transaminase pathway that synthesize meso-diaminopimelic acid, and dehydrogenase pathway that directly synthesizes racemic diaminopimelic acid without undergoing meso-diaminopimelic acid.

*Corynebacterium glutamicum* is an important industrial microorganism, and its advantage lies in its capability in fermentative production of industrial-scale amino acids. *Corynebacterium glutamicum* uses dehydrogenase pathway to synthesize L-lysine with six enzymes catalyzing the reaction, i.e., aspartate kinase (AK, coded by gene *lysC*)*,* aspartate semialdehyde dehydrogenase (ASADH, coded by *asd* gene), dihydrodipicolinate synthase (DHDPS, coded by *dapA* gene), dihydrodipicolinate reductase (DHDPR, coded by *dapB* gene), diaminopimelate dehydrogenase (DAPDH, coded by *ddh* gene) and diaminopimelate decarboxylase (DAPDC, coded by *lysA* gene). The process of L-lysine synthesis by *Corynebacterium glutamicum* using the succinylase pathway also involves four enzymes that synthesize meso-diaminopimelic acid: succinyl diaminopimelate aminotransferase (coded by *dapD* gene), tetrahydropyridine dicarboxylate succinylase (coded by *dapC* gene), succinyl diaminopimelate deacylase (coded by *dapE* gene) and diaminopimelate epimerase (coded by *dapF* gene). As well known, enhancing the expression of one or more genes in the synthesis pathway of L-lysine can effectively increase the yield of L-lysine.

Reference Document 1 discloses a *Corynebacterium* bacteria which have, in addition to at least one copy, present at the natural site (locus), of an open reading frame (ORF), gene or allele which codes for the synthesis of a protein or an RNA, in each case a second, optionally a third or a fourth copy of this open reading frame (ORF), gene or allele at in each case a second, optionally a third or a fourth site in a form of integrating into the chromosome. The *Corynebacterium* bacteria of such method can improve the gene expression level by increasing the copy number of genes, thereby increasing the yield of amino acids produced by *Corynebacterium* bacteria. However, the increase of gene copy number will reduce the stability of strain genome, thus cannot guarantee the stable and efficient production of L-lysine.

Reference Document 2 discloses a method for producing L-lysine by culturing *Escherichia coli* with L-lysine-producing capability in a medium from which L-lysine is collected, *Escherichia coli* being modified to reduce the activity or activities of one or more enzymes in the meso α,ε-diaminopimelic acid synthesis pathway, for example, 2,3,4,5-tetrahydropyridine-2,6-dicarboxylate N-succinyltransferase, succinyldiaminopimelate transaminase, succinyldiaminopimelate desuccinylase, and diaminopimelate epimerase, and *Escherichia coli* being introduced with a gene coding for diaminopimelate dehydrogenase,. This method requires increasing the expression of diaminopimelate dehydrogenase while inhibiting the enzyme activity of meso α,ε-diaminopimelic acid synthesis pathway, which suffers the problems of complicated operation process, high cost and inability to efficiently produce L-lysine.

Reference Document 3 discloses a nucleic-acid molecule that is operably ligated with a gene coding for diaminopimelate dehydrogenase, and that the diaminopimelate dehydrogenase activity can be increased to thereby increase the L-lysine yield of the strain. Although in this Document the nucleic acid molecule has higher activity compared with the wild-type promoter, it was not known whether there is a nucleic acid molecule with higher promoter activity. As well known by those skilled in the art, the higher the activity of diaminopimelate dehydrogenase is, the more favorable it is for lysine production. Therefore, the development of a promoter with higher activity will be beneficial in enhancing the expression of diaminopimelate dehydrogenase, thus having more potential for industrial applications.

### Reference Documents:

Reference Document 1: CN1748031A
Reference Document 2: CN101765659A
Reference Document 3: CN101939432A

### SUMMARY

### Technical problem

In view of the technical problem existing in the prior art, for example, *Corynebacterium* bacteria that increase the yield by increasing the copy number of genes has poor genome stability, the strain having L-lysine production capacity has defects of complicated transformation process and inability to stably and efficiently produce L-lysine. The present disclosure provides a polynucleotide having promoter activity significantly improved as compared with the promoter of wild-type *ddh* gene. By operably ligating the polynucleotide having promoter activity with a target gene, the expression intensity of the target gene can be improved while maintaining the stability of the genome.

### Solution to problem

(1) A polynucleotide having promoter activity, wherein the polynucleotide is selected from the group shown in any one of the following (i)-(ii):
   (i) a mutant of a polynucleotide having the sequence as set forth in SEQ ID NO: 9, comprising a mutated nucleotide at one or more positions in position 292 to position 300 of the sequence as set forth in SEQ ID NO: 9;
   (ii) a polynucleotide having at least 90%, optionally at least 95%, preferably at least 97%, more preferably at least 98%, and most preferably at least 99% sequence identity compared with the sequence as set forth in (i) and does not comprise the sequence as set forth in SEQ ID NO: 9;
      wherein the mutant has higher promoter activity than that of the polynucleotide having the sequence as set forth in SEQ ID NO: 9;
      and the nucleotide sequence of the mutant at position 292 to position 300 of the sequence as set forth in SEQ ID NO: 9 is not selected from ATGCATTGT.
(2) The polynucleotide having promoter activity according to (1), wherein the mutant has enhanced promoter activity of 18 folds or more as compared with the promoter activity of the polynucleotide having the sequence as set forth in SEQ ID NO: 9.
(3) The polynucleotide having promoter activity according to (1) or (2), wherein the mutant comprises mutated nucleotides at 4, 5, 6, 7, 8 or 9 positions in position 292 to position 300 of the sequence as set forth in SEQ ID NO: 9; preferably, the mutant comprises mutated nucleotides at 6, 7, 8 or 9 positions in position 292 to position 300 of the sequence as set forth in SEQ ID NO: 9.
   The polynucleotide having promoter activity according to any one of (1) to (3), wherein the nucleotide sequence at position 292 to position 300 of the polynucleotide having promoter activity is selected from any of the following groups:
   (a)ACAAAAGGT;
   (b)TCTTCATCT;
   (c)GGAAAGTAT;
   (d)TTATTATAT;
   (e)TAATCCTCT;
   (f)TCAATTTAT;
   (g)GCGCAATCT;
   (h)CAGTTCCGT;
   (i)AAGTTTTAT;
   (g)TAAATGTAT;
   (k)GGATTGTAT;
   (l)CAAACTCAT;
   (m)TACAAATCT;
   (n)TATCAGTCT;
   (o)CGAGGATAT;
   (p)CCTTGTTAT.
(5) A transcription expression cassette comprising the polynucleotide having promoter activity according to any one of (1) to (4); optionally, the transcription expression cassette further comprises a protein coding gene, the protein coding gene is operably ligated with the polynucleotide having promoter activity.
(6) A recombinant expression vector comprising the polynucleotide having promoter activity according to any one of (1) to (4) or the transcription expression cassette according to claim 5.
(7) A recombinant host cell comprising the transcription expression cassette according to (5) or the recombinant expression vector according to (6).
(8) The recombinant host cell according to (7), wherein the host cell is from genus *Corynebacterium,* genus *Brevibacterium,* genus *Arthrobacterium,* genus *Microbacterium* or genus *Escherichia*; preferably, the host cell is *Corynebacterium glutamicum*; more preferably, the host cell is *Corynebacterium glutamicum* ATCC 13032, *Corynebacterium glutamicum* ATCC 13869, *Corynebacterium glutamicum* ATCC 14067 or derivative strains thereof.
(9) Use of the polynucleotide having promoter activity according to any one of (1) to (4), the transcription expression cassette according to (5), the recombinant expression vector according to (6), or the recombinant host cell according to any one of (7) to (8) in the preparation of a reagent or kit for enhancing gene transcription level.
(10) Use of the recombinant expression vector according to (6), or the recombinant host cell according to any one of (7) to (8) in the preparation of a protein or the production of an amino acid and derivatives thereof; optionally, the amino acid and derivatives thereof are selected from one or a combination of two or more of the following: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid or derivatives of any one of the above amino acids;
   preferably, the protein is an enzyme involved in amino acid synthesis; preferably, the enzyme involved in amino acid synthesis is diaminopimelate dehydrogenase;
   preferably, the amino acid includes L-lysine and derivatives thereof, wherein the derivatives includes at least one of pentanediamine, 5-aminopentanoic acid and glutaric acid.
(11) A method for enhancing the expression of a target gene, wherein the method includes a step of operably ligating the polynucleotide having promoter activity according to any one of claims (1) to (4) with a target RNA or a target gene; optionally, the target RNA comprises at least one of tRNA and sRNA, and the target gene includes at least one of a coding gene of a protein related to the synthesis of a target compound, a coding gene of a gene expression regulatory protein and a coding gene of a protein related to membrane transport.
(12) A method for preparing a protein, wherein the transcription expression cassette according to (5), the recombinant expression vector according to (6), or the recombinant host cell according to any one of (7) to (8) is selected to express the protein; optionally, the protein is a protein related to target product synthesis, a protein related to membrane transport or a gene expression regulatory protein; optionally, the protein is an enzyme involved in the synthesis of L-lysine; optionally, the enzymes involved in the synthesis of L-lysine include one or a combination of two or more of aspartate kinase, aspartate semialdehyde dehydrogenase, aspartate ammonia lyase, dihydrodipicolinate synthase, dihydropyridinic acid reductase , succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase and pyruvate carboxylase; preferably, the protein is diaminopimelate dehydrogenase.
(13) A method for producing an amino acid and derivatives thereof, wherein the transcription expression cassette according to (5), the recombinant expression vector according to (6), or the recombinant host cell according to any one of (7) to (8) is selected to express an enzyme involved in the synthesis of the amino acid and derivatives thereof, and the enzyme involved in the synthesis of the amino acid and derivatives thereof is used to produce the amino acid and derivatives thereof; optionally, the amino acid and derivatives thereof are selected from one or a combination of two or more of: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid or derivatives of any one of the above amino acids;
   preferably, the amino acid includes L-lysine and derivatives thereof, wherein the derivatives include at least one of pentanediamine, 5-aminopentanoic acid and glutaric acid;
   preferably, the enzyme involved in the synthesis of the amino acid is diaminopimelate dehydrogenase.

### Effects

In one embodiment, the present disclosure provides a polynucleotide having promoter activity, which is a mutant of the diaminopimelate dehydrogenase gene(*ddh* gene) promoter with significantly improved promoter activity. As compared with the wild-type *ddh* gene promoter, the mutant has significantly improved promoter activity, and operably ligating it with the target gene is capable of significantly improving the expression intensity of the target gene without destroying the stability of the genome, and the target gene can be stably and efficiently expressed, and the stable and efficient production of downstream products can be realized.

In another embodiment, the present disclosure provides a transcription expression cassette, a recombinant expression vector, and a recombinant host cell, which comprise the aforesaid polynucleotide having promoter activity. In the transcription expression cassette, the recombinant expression vector and the recombinant host cell, the polynucleotide having promoter activity is operably ligated with a protein-coding gene, the expression intensity of the protein-coding gene can be improved.

In another embodiment, the present disclosure provides a method for preparing an amino acid, which can enhance the expression of the enzyme for synthesizing the amino acid by using the aforesaid polynucleotide having promoter activity, and then the amino acid can be produced stably and efficiently. When it is used in the production of L-lysine, stable and high-yield L-lysine can be obtained.

### BRIEF DESCRIPTION OF THE DRAWINGS

FIG. 1 shows a schematic diagram of pEC-XK99E-P*_{ddh}-rfp* plasmid.
FIG. 2 shows a fluorescence result displaying mutant clones grown on a culture plate, and the arrow-marked position in the figure indicates an individual clone displaying high-intensity red fluorescence.

### DETAILED DESCRIPTION

### Definitions

When used in combination with the term "comprising" in the claims and/or the specification, the word "a" or "an" may refer to "one", or refer to "one or more", "at least one", and "one or more than one".

As used in the claims and specification, the term "comprise", "have/has", "include", or "contain" is intended to be inclusive or open-ended, and does not exclude additional or unquoted elements or method steps.

Throughout the present disclosure document, the term "about" means that a value includes the standard deviation of the error of a device or method used to measure the value.

It is applicable to the content disclosed herein that the term "or" is defined only as alternatives and "and/or", but the term "or" used herein refers to "and/or" unless otherwise expressly stated to be merely alternatives or mutual exclusion between alternatives.

The selected/optional/preferred "numerical range", when used in the claims or the specification, includes not only the numerical endpoints at both ends of the range, but also all natural numbers covered between the preceding numerical endpoints with respect to these numerical endpoints.

In the present disclosure, the term "polynucleotide" refers to a polymer composed of nucleotides. Polynucleotide can be in the form of a individual fragment or a component of a larger nucleotide sequence structure, and it is derived from a nucleotide sequence separated at least once in quantity or concentration and can be recognized, manipulated and restored from the sequence and its component nucleotide sequences by standard molecular biology methods (such as using a cloning vector). When a nucleotide sequence is represented by a DNA sequence (i.e., A, T, G, C), this also includes an RNA sequence (i.e., A, U, G, C), wherein "U" replaces "T". In other words, "polynucleotide" refers to a nucleotide polymer that has been removed from other nucleotides (an individual fragment or an entire fragment), or may be a constituent part or component of a larger nucleotide structure, such as an expression vector or a polycistronic sequence. The polynucleotide includes DNA, RNA and cDNA sequences.

In the present disclosure, the term "mutation" refers to a nucleotide that comprises mutations at one or more (for example, several) positions of a polynucleotide, and maintains the promoter activity of the polynucleotide. In the present disclosure, mutation (comprising substitution, insertion and/or deletion) especially refers to substitution, which means the substitution of a nucleotide occupying a position with a different nucleotide. Deletion refers to the removal of a nucleotide occupying a certain position. Insertion refers to the addition of a nucleotide adjacent to and immediately following a nucleotide occupying a position.

In some specific embodiments, the "mutation" in the present disclosure comprises nucleotides substituted at one or more positions in position 292 to position 300 of the sequence as set forth in SEQ ID NO: 9, and does not comprise the nucleotide substituted by ATGCATTGT in position 292 to position 300 of the sequence as set forth in SEQ ID NO: 9. The mutants with nucleotides substituted at the aforesaid positions have higher promoter activity than that of the polynucleotide having the sequence as set forth in SEQ ID NO: 9.

Exemplarily, the "mutation" in the present disclosure comprises nucleotides substituted at 1, 2, 3, 4, 5, 6, 7, 8 or 9 positions in position 292 to position 300 of the sequence as set forth in SEQ ID NO: 9. In some specific embodiments, the "mutation" in the present disclosure comprises nucleotides comprising mutations at 4, 5, 6, 7, 8 or 9 positions in position 292 to position 300 of the sequence as set forth in SEQ ID NO: 9, preferably nucleotides containing mutations at 6, 7, 8 or 9 positions in position 292 to position 300 of the sequence as set forth in SEQ ID NO: 9. The mutants with the nucleotides substituted at the aforesaid positions have higher promoter activity than the mutants with nucleotides substituted at other positions.

In the present disclosure, the term "sequence identity" and "identity percentage" refer to the percentage of nucleotides or amino acids that are the same (i.e., identical) between two or more polynucleotides or polypeptides. The sequence identity between two or more polynucleotides or polypeptides can be determined by aligning the nucleotide or amino acid sequences of polynucleotides or polypeptides, scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides can be different at one position, for example, by containing a different nucleotide (i.e., substitution or mutation) or deletion of a nucleotide (i.e., nucleotide insertion or nucleotide deletion in one or two polynucleotides). Polypeptides can be different at one position, for example, by containing a different amino acid (i.e., substitution or mutation) or deletion of an amino acid (i.e., amino acid insertion or amino acid deletion in one or two polypeptides). The sequence identity can be calculated by dividing the number of positions at which nucleotide or amino acid residues are identical by the total number of nucleotide or amino acid residues in the polynucleotide or the polypeptide. For example, the identity percentage can be calculated by dividing the number of positions at which nucleotide or amino acid residues are identical by the total number of nucleotide or amino acid residues in the polynucleotide or polypeptide, and multiplying the result by 100.

In some specific embodiments, the polynucleotide having promoter activity comprises sequences having at least 90%, 91%, 92%, 93%, 94%, 95%, 96%, 97%, 98%, 99% and 100% sequence identity compared with the mutant of the polynucleotide as set forth in SEQ ID NO: 9. In the present disclosure, having a specific percentage of sequence identity means that in the mutant of the polynucleotide having the sequence as set forth in SEQ ID NO: 9 there is a mutant sequence which can maintain or improve the transcription activity of the mutant.

In the present disclosure, the term "promoter" refers to a nucleic acid molecule, which is typically located upstream of the coding sequence of the target gene, providing a recognition site for RNA polymerase, and is located upstream in the 5' direction of the transcription initiation site of mRNA. It is an untranslated nucleic acid sequence to which RNA polymerase binds and which initiates transcription of the target gene. In the synthesis of ribonucleic acid (RNA), the promoter can interact with transcription factors and regulate gene transcription and control the initiation time and degree of gene expression (transcription), and it comprises a core promoter region and regulatory region, functions like a "switch", determines the activity of the gene and then controls which kind of protein the cell starts to produce.

In the present disclosure, the term "promoter core region" refers to a nucleic acid sequence located in the promoter region of prokaryotes, and it is the core sequence region that plays the role of promoter, mainly including the -35 region, the -10 region, and the region between the -35 region and the -10 region, and the transcription initiation site, the -35 region being the recognition site of RNA polymerase, and the -10 region being the binding site of RNA polymerase.

In some specific embodiments, the polynucleotide having promoter activity in the present disclosure can be used to initiate the expression of protein-coding genes. In some other embodiments, the polynucleotide having promoter activity in the present disclosure can be used to initiate the expression of non-coding genes.

In the present disclosure, the term "expression" includes any step involving RNA production and protein production, including but not limited to transcription, post-transcriptional modification, translation, post-translational modification and secretion.

In the present disclosure, the term "protein-coding gene" refers to a DNA molecule capable of directing the synthesis of a protein by certain rules, and the process by which a protein-coding gene directing protein synthesis generally includes transcription process using double-stranded DNA as a template and translation using mRNA as a template. The protein-coding gene contains CDS sequence (Coding Sequence) directing the production of protein-coding mRNA. The protein-coding gene includes, but is not limited to, those being used to code enzymes involved in amino acid synthesis. In some embodiments, the protein-coding gene relates to coding an enzyme involved in the synthesis of L-lysine. The enzyme involved in the synthesis of L-lysine includes one or a combination of two or more of: aspartate kinase, aspartate semialdehyde dehydrogenase, aspartate ammonia lyase, dihydrodipicolinate synthetase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, and transketolase, diaminopimelate dehydrogenase and pyruvate carboxylase. The polynucleotide having promoter activity of the present disclosure is suitable for regulating the expression of the target gene and realizing the efficient production of the target product.

In the present disclosure, the term "transcription expression cassette" refers to a kind of expression element comprising a transcription regulatory element and a target gene, and uses the transcription regulatory element to regulate the expression of the target gene. In the present disclosure, the transcription regulatory element comprises a promoter, and may further comprise elements such as an enhancer, a silencer, an insulator. In the present disclosure, the target gene is specifically a protein-coding gene. "Operably ligating" of a target gene with a polynucleotide means that a polynucleotide having promoter activity is functionally ligated with a target gene to initiate and mediate the transcription of the target gene, and the operably ligating can be in any of the ways described by those skilled in the art.

In the present disclosure, the term "vector" refers to a DNA construct containing a DNA sequence operably ligated with suitable regulatory sequences, so as to express a target gene in a suitable host. "Recombinant expression vector" refers to a DNA structure used to express, for example, a polynucleotide coding a desired polypeptide. The recombinant expression vector may include, for example, i) a collection of genetic elements that have regulatory functions on gene expression, such as promoters and enhancers; ii) a structure or coding sequence transcribed into mRNA and translated into a protein; and iii) transcription subunits which appropriately transcribe and translate the start and stop sequences. The recombinant expression vector is constructed in any suitable way. The property of the vector is not critical, and any vector can be used, including plasmids, viruses, phages and transposons. Possible vectors for use in the present disclosure include, but are not limited to, chromosomal, non-chromosomal and synthetic DNA sequences, such as bacterial plasmids, phage DNA, yeast plasmids and vectors derived from combinations of plasmids and phage DNA, and DNA from viruses such as vaccinia virus, adenovirus, fowl pox virus, baculovirus, SV40 and pseudorabies virus, etc.

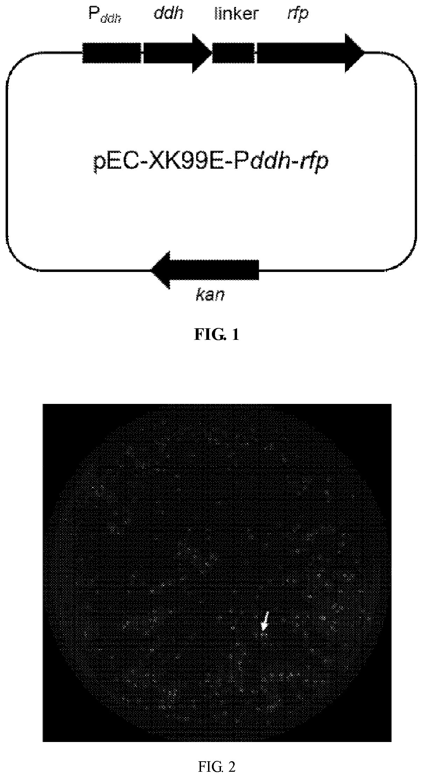

Exemplarily, the vector involved in the present disclosure is the *ddh* gene promoter intensity characterization plasmid pEC-XK99E-P*_{ddh}-rfp* constructed based on pEC-XK99E-*rfp* plasmid^{[1]}, and the map of pEC-XK99E-P*_{ddh}-rfp* plasmid is shown in FIG. 1. In FIG. 1, *P_{ddh}* represents a promoter of *ddh* gene; *ddh* represents a wild-type diaminopimelate dehydrogenase gene; linker represents a linker peptide located between *ddh* gene and *rfp* protein; *rfp* represents Red Fluorescent Protein (RFP); Kan represents Kanamycin resistant. After being transformed into a suitable host, pEC-XK99E-P*_{ddh}-rfp* can replicate and function independently of the host genome, or in some cases integrate into the host cell genome *per se.*

In the present disclosure, the term "host cell" refers to any cell type that is easily transformed, transfected, transduced, etc., with a transcription initiation element or expression vector comprising the polynucleotide of the present disclosure. The term "recombinant host cell" covers a host cell that differs from the parent cell after introduction of a transcription initiation element or a recombinant expression vector, and the recombinant host cell is specifically realized by transformation.

In the present disclosure, the term "transformation" has the meaning generally understood by those skilled in the art, i.e., the process of introducing exogenous DNA into a host. The transformation method includes any method for introducing nucleic acid into a cell, including but not limited to electroporation, calcium phosphate (CaPO₄) precipitation, calcium chloride (CaCl₂) precipitation, microinjection, polyethylene glycol (PEG) method, DEAE-dextran method, cationic liposome method and lithium acetate-DMSO method.

The host cell of the present disclosure can be a prokaryotic cell or an eukaryotic cell, as long as it is a cell to which the polynucleotide having promoter activity of the present disclosure can be introduced. In one embodiment, the host cell refers to a prokaryotic cell. Specifically, the host cell is derived from a microorganism suitable for producing amino acids by fermentation, such as genus *Corynebacterium,* genus *Brevibacterium,* genus *Arthrobacterium,* genus *Microbacterium* or genus *Escherichia.* Preferably, the host cell is *Corynebacterium glutamicum* from genus *Corynebacterium.* Among them, Corynebacterium glutamicum can be *Corynebacterium glutamicum* ATCC 13032, *Corynebacterium glutamicum* ATCC 13869, *Corynebacterium glutamicum* ATCC 14067 or derivative strains thereof.

In the present disclosure, the culture of the host cell can be carried out according to the conventional methods in art, including but not limited to well-plate culture, shaking culture, batch culture, continuous culture and fed-batch culture, etc., and various culture conditions such as temperature, time and pH value of the culture medium can be appropriately adjusted according to the actual situations.

Unless otherwise defined or clearly indicated, all technical and scientific terms in the present disclosure have the same meanings as commonly understood by those of ordinary skill in the art to which the present disclosure pertains.

### Mutant of the diaminopimelate dehydrogenase gene promoter

The present disclosure uses the promoter sequence of wild-type diaminopimelate dehydrogenase gene (*ddh*) to mutate the core promoter sequence of ddh gene, and to obtain mutants of the diaminopimelate dehydrogenase gene promoter. The promoter sequence of diaminopimelate dehydrogenase gene is as set forth in SEQ ID NO: 9, and the mutants of the diaminopimelate dehydrogenase gene promoter comprises mutated nucleotides at one or more positions in position 292 to position 300 of the sequence as set forth in SEQ ID NO: 9, and the nucleotide sequences of the mutants in position 290 to position 300 of the sequence as set forth in SEQ ID NO: 9 is not selected from ATGCATTGT.

It has been found that the mutants of the diaminopimelate dehydrogenase gene promoter is a polynucleotide having promoter activity, and the mutants of the diaminopimelate dehydrogenase gene promoter have improved promoter activity as compared with the diaminopimelate dehydrogenase gene promoter having the sequence as set forth in SEQ ID NO: 9.

In some specific embodiments, the polynucleotide having promoter activity has at least 90%, 91%, 92%, 93%, 94%, 95%, 96%, 97%, 98%, 99%, 100% sequence identity (including all ranges and percentages between these values) compared with the mutant sequence of the diaminopimelate dehydrogenase gene promoter, and does not include the sequence as set forth in SEQ ID NO: 9.

In some specific embodiments, the polynucleotide having promoter activity comprises mutated nucleotides at 4, 5, 6, 7, 8 or 9 positions in position 292 to position 300 of the sequence as set forth in SEQ ID NO: 9 and has promoter activity higher than that of the sequence as set forth in SEQ ID NO: 9, preferably mutated nucleotides at 6, 7, 8 or 9 positions in position 292 to position 300 of the sequence as set forth in SEQ ID NO: 9.

In some specific embodiments, the nucleotide sequence of position 292 to position 300 of the polynucleotide having promoter activity is selected from any of the following groups: (a) ACAAAAGGT; (b) TCTTCATCT; (c) GGAAAGTAT; (d) TTATTATAT; (e) TAATCCTCT; (f) TCAATTTAT; (g) GCGCAATCT; (h) CAGTTCCGT; (i) AAGTTTTAT; (g) TAAATGTAT; (k) GGATTGTAT; (l) CAAACTCAT; (m) TACAAATCT; (n) TATCAGTCT; (o) CGAGGATAT; (p) CCTTGTTAT. The polynucleotide having promoter activity comprises sequences as set forth in any one of SEQ ID NOs: 10-25. As compared with the diaminopimelate dehydrogenase gene promoter having the sequence as set forth in SEQ ID NO: 9, the mutants have promoter activity enhanced by 18 folds or more, preferably 19 folds or more, preferably 20 folds or more, preferably 22 folds or more, preferably 23 folds or more, preferably 24 folds or more, preferably 25 folds or more, preferably 26 folds or more, more preferably 27 folds or more, more preferably 28 folds or more, more preferably 30 folds or more and more preferably 31 folds or more.

### Construction of recombinant expression vector

In some specific embodiments, a recombinant vector comprising the diaminopimelate dehydrogenase gene promoter is first constructed, and then the core promoter region of *ddh* gene is mutated to obtain a recombinant expression vector comprising a polynucleotide having promoter activity.

For the construction of the recombinant vector, primers ddh-F and ddh-R are designed according to the published genome information of *Corynebacterium glutamicum,* and the genome of *Corynebacterium glutamicum* is subject to PCR amplification with ddh-F and ddh-R to obtain the promoter sequence *of ddh* gene.

Primers pEC-F and pEC-R are designed by using information of pEC-XK99E-*rfp*^{[1]} plasmid. With the pEC-XK99E-*rfp* plasmid as a template, primers pEC-F and PEC-R are used to perform amplification to obtain DNA fragments of the pEC-XK99E skeleton, linker peptides and red fluorescent protein.

The amplified fragments resulting from primers *ddh-*F and *ddh-*R and the amplified fragments resulting from primers pEC-F and pEC-R are recombinantly ligated, to obtain a recombinant expression vector linked with a polynucleotide having promoter activity (i.e., *ddh* gene promoter mutant sequence).

The specific source of *Corynebacterium glutamicum* is *Corynebacterium glutamicum* ATCC 13032 (Gene ID: 2830649).

### Production process of amino acids

(1) In the present disclosure, the polynucleotide having promoter activity is operably ligated with the coding gene of an enzyme involved in amino acid synthesis, to obtain a recombinant expression vector capable of synthesizing the enzyme involved in amino acid synthesis, and the recombinant expression vector is used to transform the host cell to obtain a recombinant host cell.
(2) The recombinant host cell is subject to fermentation culture, and amino acids are collected from the recombinant host cell or the culture broths of the recombinant host cell to complete the production process of amino acids.

In the aforesaid production process, since the polynucleotide has improved promoter activity, in the recombinant host cell, the transcription activity of the coding gene of the enzyme involved in amino acid synthesis is increased, and the expression level of the enzyme involved in amino acid synthesis is increased, thereby the yield of amino acids is significantly increased.

Regarding the produced amino acids, the amino acids include L-lysine and derivatives thereof. Optionally, the amino acid is selected from one or a combination of two or more of the following: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid or derivatives of any one of the aforesaid amino acids. Preferably, the derivatives include at least one of pentanediamine, 5-aminopentanoic acid and glutaric acid.

As regards the enzyme involved in amino acid synthesis, the enzyme involved in amino acid synthesis is that involved in L-lysine synthesis; preferably, the enzyme involved in amino acid synthesis is diaminopimelate dehydrogenase. The polynucleotide having promoter activity can significantly increase the transcription activity of diaminopimelate dehydrogenase and significantly increase the expression level of diaminopimelate dehydrogenase.

In some embodiments, the host cell in the present disclosure can be any type of strain having the capability for producing a target product, including wild-type strains and recombinant strains. Exemplarily, the host cell is derived from microorganisms suitable for fermentative production of a target product such as an amino acid and derivatives thereof, for example *Enterobacterium, Corynebacterium, Brevibacterium, Arthrobacterium, Microbacterium,* etc.

In some preferable embodiments, the host cell is *Enterobacterium* or *Corynebacterium,* more preferably *Corynebacterium glutamicum,* including but not limited to *Corynebacterium glutamicum* ATCC 13032, *Corynebacterium glutamicum* ATCC 13869, *Corynebacterium glutamicum* B253, *Corynebacterium glutamicum* ATCC 14067, and derivative strains producing L-amino acids prepared from the above strains.

In some specific embodiments, the host cell is *Corynebacterium glutamicum,* an important strain for producing L-lysine. After modification of *Corynebacterium glutamicum* using a polynucleotide, a transcription expression cassette or a recombinant expression vector having promoter activity, the expression level of the enzyme involved in L-lysine synthesis in *Corynebacterium glutamicum* is significantly increased, specifically the expression level of diaminopimelate dehydrogenase is significantly increased, resulting in a much higher capacity for fermentative production of L-lysine in *Corynebacterium glutamicum.*

In some specific embodiments, the recombinant host cell is *Corynebacterium glutamicum* modified as follows: 1) a T311I mutated coding sequence is introduced into the coding gene of aspartate kinase in *Corynebacterium glutamicum;* 2) the core region from position 279 to position 317 of pyruvate carboxylase gene promoter in *Corynebacterium glutamicum* is CGGGCCTTTGAAGACATTATTAGATTATTATTATTATTATTAG; 3) a polynucleotide having promoter activity is operably ligated with the gene coding diaminopimelate dehydrogenase, and then introduced into *Corynebacterium glutamicum. Corynebacterium glutamicum* modified as the above method is a high-yield strain of L-lysine.

Specifically, the coding genes of aspartate kinase, pyruvate carboxylase and diaminopimelate dehydrogenase can be isolated and inserted into an expression vector for transformation production, where in the host cell the expression vector can replicate and express the enzymes independently of the host cell, or in some cases integrate into the host cell genome. In the art, methods for manipulating microorganisms are known, as explained in publications such as Current Protocols in Molecular Biology (Online ISBN: 9780471142720, John Wiley and Sons, Inc.), Microbial Metabolic Engineering: Methods and Protocols (Qiong Cheng Ed.,Springer) and Systems Metabolic Engineering: Methods and Protocols (Hal S.Alper Ed., Springer).

In some other embodiments, the host cell can also be other kinds of amino acid producing strains. The "amino acid producing strain" in the present disclosure refers to a strain that can produce amino acids and accumulate amino acids when bacteria are cultured in a culture medium, or secrete amino acids into the culture medium, i.e., a strain that can obtain extracellular free amino acids. For example, it can be a naturally occurring amino acid producing strain or an engineered amino acid producing strain obtained by genetic modification.

Exemplarily, the host cell is a lysine-producing host cell. In some embodiments, the lysine-producing host cell may contain, but is not limited to, one or more genes selected from the following that are attenuated or reduced in expression:
a. *adhE* gene coding ethanol dehydrogenase;
b. *ackA* gene coding acetate kinase;
c. *pta* gene coding phosphate acetyltransferase;
d. *IdhA* gene coding lactate dehydrogenase;
e*. focA* gene coding formate transporter;
f. *pflB* gene coding pyruvate formate lyase;
g. *poxB* gene coding pyruvate oxidase;
h. *thrA* gene coding aspartate kinase I/ homoserine dehydrogenase I bifunctional enzyme;
i. *thrB* gene coding homoserine kinase;
j. *ldcC* gene coding lysine decarboxylase; and
h. *cadA* gene coding lysine decarboxylase.

In some embodiments, the lysine-producing host cell may contain, but is not limited to, one or more genes selected from the following that are enhanced or overexpressed:
a. *dapA* gene coding dihydrodipyridine synthase that relieves feedback inhibition of lysine;
b. *dapB* gene coding dihydrodipicolinate reductase;
c. *ddh* gene coding diaminopimelate dehydrogenase;
d. *dapD* coding tetrahydrodipicolinate succinylase and dapE coding succinyl diaminopimelate deacylase;
e. *asd* gene coding aspartate-semialdehyde dehydrogenase;
f. *ppc* gene coding phosphoenolpyruvate carboxylase;
g. *pntAB* gene coding nicotinamide adenine dinucleotide transhydrogenase;
i. *lysE* gene coding lysine transport protein.

Exemplarily, the host cell is a threonine-producing host cell. In some embodiments, the threonine-producing host cell is a strain that expresses aspartate kinase *LysC* gene that relieves feedback inhibition on the basis of *Corynebacterium glutamicum* ATCC 13032. In some other embodiments, the threonine-producing host cell can also be other strains having threonine-producing ability.

In some embodiments, the lysine-producing host cell may contain, but is not limited to, one or more genes selected from the following that are enhanced or overexpressed:
a. *thrABC* gene coding threonine operon;
b. *hom* gene coding homoserine dehydrogenase that relieves feedback inhibition;
c. *gap* gene coding glyceraldehyde-3-phosphate dehydrogenase;
d. *pyc* gene coding pyruvate carboxylase;
e. *mqo* gene coding malate: quinone oxidoreductase;
f. *tkt* gene coding transketolase;
g. *gnd* gene coding 6-phosphogluconate dehydrogenase;
h. *thrE* gene coding threonine exporter;
i. *eno* gene coding enolase.

Exemplarily, the host cell is an isoleucine-producing host cell. In some embodiments, the isoleucine-producing host cell is a strain that produces L-isoleucine by substituting alanine for the amino acid at position 323of the ilvA gene of L-threonine dehydratase. In some other embodiments, the isoleucine-producing host cell can also be other types of strains having isoleucine-producing capability.

Exemplarily, the host cell is an O-acetylhomoserine-producing host cell. In some embodiments, the O-acetylhomoserine-producing host cell is a strain that produces O-acetylhomoserine by inactivating O-acetylhomoserine (thiol)-lyase. In some other embodiments, the O-acetylhomoserine-producing host cell can also be other types of strains having O-acetylhomoserine production ability.

Exemplarily, the host cell is a methionine-producing host cell. In some embodiments, the methionine-producing host cell is a strain that produces methionine by inactivating transcription regulators of methionine and cysteine. In some other embodiments, the methionine-producing host cell can also be other types of strains having methionine-producing ability.

In some specific embodiments, the culture conditions of recombinant host cells are as follows: at first, *Corynebacterium glutamicum* is inoculated into TSB liquid medium and cultured for 8 hours, and the culture is inoculated as a seed into a 24-well plate of 800µl fermentation medium/well and, with an initial OD controlled to be about 0.1, cultured at 30°C for 17 hours at a rotation speed of the plate shaker of 800rpm.

Ingredients of the TSB liquid medium are as follows: glucose, 5g/L; yeast powder, 5g/L; soy peptone, 9g/L; Urea, 3g/L; succinic acid, 0.5g/L; K₂HPO₄·3H₂O, 1g/L; MgSO₄·7H₂O, 0.1g/L; biotin, 0.01mg/L; vitamin B1,0.1mg/L; MOPS, 20g/L.

Ingredients of the fermentation medium are as follows: glucose, 80g/L; yeast powder, 1g/L; soybean peptone, 1g/L; NaCl, 1g/L; Ammonium sulfate, 1g/L; Urea, 10g/L; K₂HPO₄·3H₂O, 1g/L; MgSO₄·7H₂O, 0.45g/L; FeSO₄·7H₂O, 0.05g/L; biotin, 0.4mg/L; vitamin B1, 0.1mg/L; MOPS, 40g/L; initial pH 7.2.

In some specific embodiments, amino acids can be recovered from recombinant host cells or culture broths of recombinant cells by methods commonly used in the art, including but not limited to filtration, anion exchange chromatography, crystallization and HPLC.

### Examples

Other objects, features and advantages of the present disclosure will become apparent from the following detailed description. However, it should be understood that the detailed description and specific examples (although indicating specific embodiments of the present disclosure) are given for explanatory purpose only, because various variations and modifications within the spirit and scope of the present disclosure will become obvious to those skilled in the art upon reading the detailed description.

The experimental techniques and methods used in the examples are conventional technical methods unless otherwise specified. For example, the experimental methods for which specific conditions are not indicated in the following examples are usually in accordance with conventional conditions, such as those described in Molecular Cloning: Laboratory Manual (New York: Cold Spring Harbor Laboratory Press, 1989), by Sambrook et al., or those suggested by manufacturers. Unless otherwise specified, the materials and reagents used in the examples can be obtained through formal commercial channels.

### Example 1. Construction of intensity characterization plasmid of Corynebacterium glutamicum ddh gene promoter

In order to characterize the intensity of the *ddh* gene promoter of *Corynebacterium glutamicum,* the present example first constructed a characterization vector, ligating the *ddh* gene promoter and a *ddh* gene fragment based on the skeleton of the pEC-XK99E plasmid, and the *ddh* gene, linker peptide and a red fluorescent protein (rfp) gene were expressed using the *ddh* gene promoter. The details are as follows.
(1) Primers *ddh-*F and *ddh-*R were designed according to the published genome sequence of *Corynebacterium glutamicum* ATCC13032 (Gene ID:2830649) and the annotation information *of ddh* gene, using ATCC 13032 genome as a template, a *ddh* gene promoter and a *ddh* gene fragment were obtained by PCR amplification. The nucleotide sequence of the amplified fragment is as set forth in SEQ ID NO: 34.
(2) DNA fragments of pEC-XK99E plasmid skeleton, linker peptide and red fluorescent protein gene were amplified using PEC-XK99E-*rfp* plasmid as a template, and pEC-F and pEC-R as primers. The nucleotide sequence of the amplified fragment is set forth in SEQ ID NO: 35.

The amplified genome fragment of *Corynebacterium glutamicum* ATCC13032 and the amplified plasmid fragment of pEC-XK99E-*rfp* were cloned and ligated via Vazyme's One Step Cloning Kit to obtain a pEC-XK99E-P*_{ddh}-rfp* characterization vector, of which the plasmid map is shown in FIG. 1. The sequences of the primers used above are shown in Table 1, and the sequence information of the linker peptide is shown in Table 2.

**Table 1**

| Primers | Nucleotide sequence | SEQ ID NO. |
|---|---|---|
| *ddh-F* | CCTGATGCGGTATTTTCTCCGTGCGTGGCGAGTTTT ACAAAG | SEQ ID NO: 1 |
| *ddh-R* | GACGTCGCGTGCGATCAGATC | SEQ ID NO: 2 |
| pEC-F | ATCTGATCGCACGCGACGTCGGCGGTGGCTCTGGA GGTGGTGGGTCCGGCGGTGGCTCTGCTTCCTCCGA AGACGTTATCAAAG | SEQ ID NO: 3 |
| pEC-R | GGAGAAAATACCGCATCAGGC | SEQ ID NO: 4 |

**Table 2**

| | Amino acid sequence | Nucleotide sequence coding linker peptide |
|---|---|---|
| Linker peptide | GGGSGGGGSGGGS (SEQ ID NO: 36) | GGCGGTGGCTCTGGAGGTGGTGGGTCCGGCGGTGGCTCT (SEQ ID NO: 37) |

### Example 2. Screening and intensity characterization of mutants of Corynebacterium glutamicum ddh gene promoter

### (1) Construction of mutant library of Corynebacterium glutamicum ddh gene promoter

In the present example, the core region "TGATGAAAGAGATGTCCCTGAA TCATCATCTAAGTATGCATCTCGGTAAGCTCGACCAGG" of the *Corynebacterium glutamicum ddh* gene promoter was mutated, wherein the underlined parts are main sequences of the -35 region and the -10 region of the promoter, respectively. In the present example, the mutation
"TGATGAAAGAGATGTCCCTGAATCATCATCTAAGTNNNNNNNNNGGTAAGCTCGA CCAGG" was carried out at a corresponding position of the aforesaid core region, two fragments of the plasmid were amplified by using *ddh-*M1*, ddh-*M2*, ddh-*M3 and *ddh-*M4 primers, respectively, and cloned and ligated via Vazyme's One Step Cloning Kit. All the obtained clones were collected and plasmids were extracted to obtain a library of *ddh* gene promoter mutants. In order to compare with the *ddh* promoter mutants already published in the prior art, we constructed a same *ddh* promoter mutant as disclosed in Reference Document 3 (CN101939432A) on the basis of pEC-XK99E-P*_{ddh}-rfp* plasmid. Specifically, the sequence of the promoter core region is "TGTGTGAAAAGAGAATGTCCGATCATAAGTATGCATtgtGGTAAGCTCCGAGG", wherein the underlined part is the mutant sites disclosed by Reference Document 3. *Corynebacterium glutamicum* ATCC13032 was transformed with the aforesaid library, the mutant pEC-XK99E- *P_{ddh}-x-rfp* in the prior art and the wild type control pEC-XK99E-P*_{ddh}-rfp* obtained in Example 1, respectively, and coated on a TSB plate. The plate, on which hundreds of clones were grown, was fluorescence photographed with a fluorescence imaging system, and mutants with improved expression intensity were preliminarily screened according to the fluorescence brightness of the clones. FIG. 2 shows a fluorescence photograph of mutant clones grown on the culture plate, in which the arrow-marked position is an individual clone displaying high intensity red fluorescence. Ingredients (g/L) of the TSB plate medium are as follows: glucose, 5g/L; yeast powder, 5g/L; soy peptone, 9g/L; Urea, 3g/L; succinic acid, 0.5g/L; K₂HPO₄·3H₂O, 1g/L; MgSO₄·7H₂O, 0.1g/L; biotin, 0.01mg/L; vitamin B1, 0.1mg/L; MOPS, 20g/L; agar powder, 15g/L. In the present example, more than 10,000 clones were preliminarily screened, and about 20 mutants with significantly enhanced fluorescence intensity were obtained. The sequences of the primers used above are shown in Table 3.

**Table 3**

| Primers | Nucleotide sequence | SEQ ID NO. |
|---|---|---|
| *ddh*-M1 | CCCTGAATCATCATCTAAGTNNNNNNNNNGGT AAGCTCGACCAGGACAGTG | SEQ ID NO: 5 |
| *ddh*-M2 | AACCTTCCATACGAACTTTGAAACG | SEQ ID NO: 6 |
| *ddh*-M3 | CAAAGTTCGTATGGAAGGTTCCG | SEQ ID NO: 7 |
| *ddh*-M4 | ACTTAGATGATGATTCAGGGAC | SEQ ID NO: 8 |

### (2) Intensity characterization of the library of Corynebacterium glutamicum ddh gene promoter mutants

All mutants with enhanced fluorescence intensity observed on the above plates were cultured in a 96-well plate to characterize the intensity of promoters. Ingredients (g/L) of the TSB liquid medium are as follows: glucose, 5g/L; yeast powder, 5g/L; soy peptone, 9g/L; Urea, 3g/L; succinic acid, 0.5g/L; K₂HPO₄·3H₂O, 1g/L; MgSO₄·7H₂O, 0.1g/L; biotin, 0.01mg/L; vitamin B1, 0.1mg/L; MOPS, 20g/L. The strains resulting from the screening, the wild-type control and the prior-art control were inoculated with toothpicks into a 96-well plate containing 200µl TSB liquid culture medium in each well, with three parallel strains for each strain. At a rotation speed of the plate shaker of 800rpm, after culturing at 30°C for 24 hours, the fluorescence intensities of the strains were detected, and the strains with improved fluorescence intensity as compared with the wild-type control were sequenced. Some promoter mutants had the same sequence, and finally 16 different promoter mutants with significantly improved expression intensity were successfully obtained, and the results are shown in Table 4, in which the promoters obtained in the present disclosure are sequentially numbered as *P_{ddh}-1* to *P_{ddh}-16.* The promoter activities of *P_{ddh}-*1 to P*_{ddh}*-16 were increased by about 18 to 31 folds, providing abundant elements for modifying the expression of genes such as *ddh* etc.

The fluorescence intensity of *ddh* promoter mutant (P*_{ddh}*-x) of Reference document 3 was detected by the above method, and the result is shown in Table 4. The activity of P*_{ddh}*-x was only 15 folds increased as compared with that of the wild-type promoter, and the expression intensities of the promoter of the present invention were improved by 15-101% as compared with that of the promoter disclosed in the prior art, and is therefore a substantial progress.

**Table 4**

| Promoter No. | Fluorescence intensity (RFU/OD₆₀₀) | Sequence of promoter mutation region | Sequence No. of the complete promoter sequence |
|---|---|---|---|
| Wild type | 609 | ATGCATCTC | SEQ ID NO: 9 |
| P*_{ddh}*-1 | 11134 | ACAAAAGGT | SEQ ID NO: 10 |
| P*_{ddh}*-2 | 11215 | TCTTCATCT | SEQ ID NO: 11 |
| P*_{ddh}*-3 | 11247 | GGAAAGTAT | SEQ ID NO: 12 |
| P*_{ddh}*-4 | 11264 | TTATTATAT | SEQ ID NO: 13 |
| P*_{ddh}*-5 | 11366 | TAATCCTCT | SEQ ID NO: 14 |
| P*_{ddh}*-6 | 11367 | TCAATTTAT | SEQ ID NO: 15 |
| P*_{ddh}*-7 | 11421 | GCGCAATCT | SEQ ID NO: 16 |
| P*_{ddh}*-8 | 11557 | CAGTTCCGT | SEQ ID NO: 17 |
| P*_{ddh}*-9 | 11792 | AAGTTTTAT | SEQ ID NO: 18 |
| P*_{ddh}*-10 | 11808 | TAAATGTAT | SEQ ID NO: 19 |
| P*_{ddh}*-11 | 11853 | GGATTGTAT | SEQ ID NO: 20 |
| P*_{ddh}*-12 | 12396 | CAAACTCAT | SEQ ID NO: 21 |
| P*_{ddh}*-13 | 12923 | TACAAATCT | SEQ ID NO: 22 |
| P*_{ddh}*-14 | 13799 | TATCAGTCT | SEQ ID NO: 23 |
| P*_{ddh}*-15 | 13860 | CGAGGATAT | SEQ ID NO: 24 |
| P*_{ddh}*-16 | 19417 | CCTTGTTAT | SEQ ID NO: 25 |
| P*_{ddh}*-x | 9669 | ATGCATTGT | SEQ ID NO: 38 |

### Example 3. Application of Corynebacterium glutamicum ddh gene promoter mutants in lysine production

### (1) Construction of recombinant vectors of Corynebacterium glutamicum ddh gene promoter mutants

According to the reported genome sequence of *Corynebacterium glutamicum* ATCC13032, the upstream and downstream homologous arms of *P_{ddh}-1,* P*_{ddh}*-10 and P*_{ddh}*-16 promoter mutations were subjected to PCR amplification using ATCC13032 genome as a template, and using *ddh-UF*/*ddh*-UR and *ddh-*DF1/*ddh-*DR*, ddh-*UF/*ddh-*UR and *ddh-DF10*/*ddh-DR, ddh-UF*/*ddh-UR* and *ddh-*DF16/*ddh-*DR as primers, respectively; meanwhile, the skeleton of pK18mobsacB (GenBank:FJ437239.1) was amplified using pK18-1/2 as primers. The above two groups of PCR fragments were recovered and then ligated via Vazyme's One Step Cloning Kit, and recombinant vectors pK18-P*_{ddh}*-1, pK18-P*_{ddh}*-10 and pK18-P*_{ddh}*-16 with mutated promoters were obtained, respectively. The sequences of the primers used above are listed in Table 5.

**Table 5**

| Primers | Nucleotide sequence | SEQ ID NO. |
|---|---|---|
| *ddh*-UF | CAGGAAACAGCTATGACATGTTCCAGCCATCGCCAAATAAG | SEQ ID NO: 26 |
| *ddh*-UR | ACTTAGATGATGATTCAGGGAC | SEQ ID NO: 27 |
| *ddh*-DF1 | CCCTGAATCATCATCTAAGTACAAAAGGTGGTAAGCTCGACCAG GACAGTG | SEQ ID NO: 28 |
| *ddh*-DF10 | CCCTGAATCATCATCTAAGTTAAATGTATGGTAAGCTCGACCAGG ACAGTG | SEQ ID NO: 29 |
| *ddh-*DF16 | CCCTGAATCATCATCTAAGTCCTTGTTATGGTAAGCTCGACCAGG ACAGTG | SEQ ID NO: 30 |
| *ddh*-DR | TGTAAAACGACGGCCAGTGCTGTACTGGACTGCCTTTTGAACG | SEQ ID NO: 31 |
| pK18-1 | GCACTGGCCGTCGTTTTAC | SEQ ID NO: 32 |
| pK18-2 | CATGTCATAGCTGTTTCCTGTGTG | SEQ ID NO: 33 |

### (2) Construction of pyc gene promoter mutant of Corynebacterium glutamicum lysine producing strain

*Corynebacterium glutamicum* lysine-producing strain SCgL37 (a strain in which a T311I (base mutated from ACC to ATC) mutated coding sequence is introduced into the coding gene of aspartate kinase in *Corynebacterium glutamicum* ATCC13032, and the core region at position 279 to position 317 of pyruvate carboxylase gene promoter is mutated to CGGGCCTTTGATGAGAGAGACATTATATATATATATATATATATATATAG) was transformed by the recombinant vectors pK18-P*_{ddh}*-1, pK18-P*_{ddh}-*10, and pK18-P_{ddh}-16 constructed above, respectively, and coated on a LBHIS solid medium containing 5g/L glucose and 25µg/mL kanamycin, and cultured at 30°C to obtain the first recombinant transformant. The correct primary recombinant transformants were inoculated into a LB medium containing 5g/L glucose, cultured overnight, and diluted and coated on LB solid medium plates to which 100g/L sucrose was added, respectively, to undergo screening, and *ddh* promoter mutant strains SCgL38, SCgL39 and SCgL40 were obtained, respectively.

### (3) Evaluation of L-lysine production capacity of ddh gene promoter mutants of Corynebacterium glutamicum lysine-producing strains

In order to test the effect of *ddh* promoter mutation in *Corynebacterium glutamicum* on L-lysine production, fermentation tests were carried out on SCgL37, SCgL38, SCgL39 and SCgL40 strains, respectively. Ingredients of the fermentation medium is as follows: glucose, 80g/L; yeast powder, 1g/L; soybean peptone, 1g/L; NaCl, 1g/L; ammonium sulfate, 1g/L; Urea, 10g/L; K₂HPO₄·3H₂O, 1g/L; MgSO₄·7H₂O, 0.45g/L; FeSO₄·7H₂O, 0.05g/L; biotin, 0.4mg/L; vitamin B1, 0.1mg/L; MOPS, 40g/L; initial pH7.2. Firstly, the strains were inoculated into TSB liquid medium to undergo culture for 8 hours, and the cultures were inoculated as seeds into a 24-well plate containing 800µl of fermentation medium in each well. The initial OD₆₀₀ was controlled at about 0.1, and the culture was carried out at 30°C for 17 hours at a rotation speed of the plate shaker of 800rpm, with three parallel strains for each strain. After the fermentation, the L-lysine yield and glucose consumption were measured, and the conversion rates of glucose from glucose to L-lysine were calculated. The results are shown in Table 6, which shows that the strains with mutated *ddh* promoters had increased lysine yield and increased glucose-acid conversion rate, and the increase was more significant with the increase of the promoter intensity. The above results indicate that the mutants with enhanced expression intensity of *ddh* promoter can be applied to L-lysine production.

**Table 6**

| Strains | Lysine yield (g/L) | Conversion rate (%) |
|---|---|---|
| SCgL37 | 2.97±0.06 | 5.41±0.04 |
| SCgL38 | 3.47±0.21 | 6.47±0.57 |
| SCgL39 | 3.53±0.21 | 7.05±1.09 |
| SCgL40 | 3.60±0.36 | 7.49±1.26 |

Since the synthesis of downstream products of L-lysine all depends on the reaction steps catalyzed by diaminopimelate dehydrogenase, the yield of downstream products can also be increased by enhancing the expression of *ddh* gene by the mutants of *ddh* gene promoter of the present disclosure. Therefore, the technical solution provided by the present disclosure can also be used for the production of downstream products of L-lysine, such as pentanediamine, 5-aminovaleric acid, glutaric acid, etc.

All technical features disclosed in this specification can be combined in any way of combination. Each feature disclosed in this specification can also be replaced by another feature having the same, equal or similar function. Therefore, unless otherwise specified, each feature disclosed herein is merely an example of a series of equal or similar features.

In addition, according to the above description of the present disclosure, those skilled in the art can easily understand the key features of the present disclosure, and many modifications can be made to the invention to adapt to various use purposes and conditions without departing from the spirit and scope of the present disclosure, and therefore such modifications are also intended to fall within the scope of the appended claims.

### Reference:

[1] Wang, YC et al. Screening efficient constitutive promoters in Corynebacterium glutamicum based on time-series transcriptome analysis. Chinese Journal of Biotechnology, 2018, 34(11):1760 - 1771

## Claims

1. A polynucleotide having promoter activity, wherein the polynucleotide is selected from the group shown in any one of the following (i)-(ii):
(i) a mutant of a polynucleotide having the sequence as set forth in SEQ ID NO: 9, comprising a mutated nucleotide at one or more positions in position 292 to position 300 of the sequence as set forth in SEQ ID NO: 9;
(ii) a polynucleotide having at least 90%, optionally at least 95%, preferably at least 97%, more preferably at least 98%, and most preferably at least 99% sequence identity compared with the sequence as set forth in (i) and does not comprise the sequence as set forth in SEQ ID NO: 9;
wherein the mutant has higher promoter activity than that of the polynucleotide having the sequence as set forth in SEQ ID NO: 9; and
the nucleotide sequence of the mutant at position 292 to position 300 of the sequence as set forth in SEQ ID NO: 9 is not selected from ATGCATTGT.

2. The polynucleotide having promoter activity according to claim 1, wherein the mutant has enhanced promoter activity of 18 folds or more as compared with the polynucleotide having the sequence as set forth in SEQ ID NO: 9.

3. The polynucleotide having promoter activity according to claim 1 or 2, wherein the mutant comprises mutated nucleotides at 4, 5, 6, 7, 8 or 9 positions in position 292 to position 300 of the sequence as set forth in SEQ ID NO: 9; preferably, the mutant comprises mutated nucleotides at 6, 7, 8 or 9 positions in position 292 to position 300 of the sequence as set forth in SEQ ID NO: 9.

4. The polynucleotide having promoter activity according to any one of claims 1 to 3, wherein the nucleotide sequence at position 292 to position 300 of the polynucleotide having promoter activity is selected from any of the following groups:
(a)ACAAAAGGT;
(b)TCTTCATCT;
(c)GGAAAGTAT;
(d)TTATTATAT;
(e)TAATCCTCT;
(f)TCAATTTAT;
(g)GCGCAATCT;
(h)CAGTTCCGT;
(i)AAGTTTTAT;
(g)TAAATGTAT;
(k)GGATTGTAT;
(l)CAAACTCAT;
(m)TACAAATCT;
(n)TATCAGTCT;
(o)CGAGGATAT;
(p)CCTTGTTAT.

5. A transcription expression cassette comprising the polynucleotide having promoter activity according to any one of claims 1 to 4; optionally, the transcription expression cassette further comprises a protein coding gene, the protein coding gene is operably ligated with the polynucleotide having promoter activity.

6. A recombinant expression vector comprising the polynucleotide having promoter activity according to any one of claims 1 to 4 or the transcription expression cassette according to claim 5.

7. A recombinant host cell comprising the transcription expression cassette according to claim 5 or the recombinant expression vector according to claim 6.

8. The recombinant host cell according to claim 7, wherein the host cell is from genus *Corynebacterium,* genus *Brevibacterium,* genus *Arthrobacterium,* genus *Microbacterium* or genus *Escherichia*; preferably, the host cell is *Corynebacterium glutamicum;* more preferably, the host cell is *Corynebacterium glutamicum* ATCC 13032, *Corynebacterium glutamicum* ATCC 13869, *Corynebacterium glutamicum* ATCC 14067 or derivative strains thereof.

9. Use of the polynucleotide having promoter activity according to any one of claims 1 to 4, the transcription expression cassette according to claim 5, the recombinant expression vector according to claim 6, or the recombinant host cell according to any one of claims 7 to 8 in the preparation of a reagent or kit for enhancing gene transcription level.

10. Use of the recombinant expression vector according to claim 6, or the recombinant host cell according to any one of claims 7 to 8 in the preparation of a protein or the production of an amino acid and derivatives thereof;
optionally, the amino acid and derivatives thereof are selected from one or a combination of two or more of the following: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid or derivatives of any one of the above amino acids;
preferably, the protein is an enzyme involved in the synthesis of an amino acid and derivatives thereof; preferably, the enzyme involved in the synthesis of an amino acid is diaminopimelate dehydrogenase;
preferably, the amino acid comprises L-lysine and derivatives thereof, wherein the derivatives includes at least one of pentanediamine, 5-aminopentanoic acid and glutaric acid.

11. A method for enhancing the expression of a target gene, wherein the method comprises a step of operably ligating the polynucleotide having promoter activity according to any one of claims 1 to 4 with a target RNA or a target gene; optionally, the target RNA comprises at least one of tRNA and sRNA, and the target gene comprises at least one of a coding gene of a protein related to the synthesis of a target compound, a coding gene of a gene expression regulatory protein and a coding gene of a protein related to membrane transport.

12. A method for preparing a protein, wherein the transcription expression cassette according to claim 5, the recombinant expression vector according to claim 6, or the recombinant host cell according to any one of claims 7 to 8 is selected to express the protein; optionally, the protein is a protein related to target product synthesis, a protein related to membrane transport or a gene expression regulatory protein; optionally, the protein is an enzyme involved in the synthesis of L-lysine; optionally, the enzymes involved in the synthesis of L-lysine include one or a combination of two or more of aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase and pyruvate carboxylase; preferably, the protein is diaminopimelate dehydrogenase.

13. A method for producing an amino acid and derivatives thereof, wherein the transcription expression cassette according to claim 5, the recombinant expression vector according to claim 6, or the recombinant host cell according to any one of claims 7 to 8 is selected to express an enzyme involved in the synthesis of the amino acid and derivatives thereof, and the enzyme involved in the synthesis of the amino acid and derivatives thereof is used to produce the amino acid and derivatives thereof; optionally, the amino acid and derivatives thereof are selected from one or a combination of two or more of: proline, hydroxyproline, lysine, glutamic acid, arginine, ornithine, glutamine, threonine, glycine, alanine, valine, leucine, isoleucine, serine, cysteine, methionine, aspartic acid, asparagine, histidine, phenylalanine, tyrosine, tryptophan, 5-aminolevulinic acid or derivatives of any one of the above amino acids;
preferably, the amino acid includes L-lysine and derivatives thereof, wherein the derivatives include at least one of pentanediamine, 5-aminopentanoic acid and glutaric acid;
preferably, the enzyme involved in the synthesis of the amino acid is diaminopimelate dehydrogenase.
